(19) Europäisches Patentamt
European Patent Office
Office européen des brevets

(11) **EP 1 529 513 B1**

(12) **FASCICULE DE BREVET EUROPEEN**

(45) Date de publication et mention
de la délivrance du brevet:
**05.03.2008 Bulletin 2008/10**

(51) Int Cl.:
*A61Q 1/02* (2006.01)          *A61K 8/04* (2006.01)
*A61K 8/06* (2006.01)          *A61K 8/19* (2006.01)
*A61K 8/29* (2006.01)          *A61K 8/39* (2006.01)
*A61K 8/55* (2006.01)

(21) Numéro de dépôt: **04292494.4**

(22) Date de dépôt: **20.10.2004**

(54) **Composition de maquillage comprenant une émulsion comprenant des tensioactifs non-ioniques, des tensioactifs ioniques, et des particules solides**

Dekorative kosmetische Zusammensetzung auf Basis einer Emulsion mit nichtionischem und ionischem Emulgator und festen Partikeln

Make-up composition on emulsion basis comprising non-ionic and ionic surface active agent and solid particles

(84) Etats contractants désignés:
**AT BE BG CH CY CZ DE DK EE ES FI FR GB GR HU IE IT LI LU MC NL PL PT RO SE SI SK TR**

(30) Priorité: **07.11.2003 FR 0350810**

(43) Date de publication de la demande:
**11.05.2005 Bulletin 2005/19**

(73) Titulaire: **L'ORÉAL**
**75008 Paris (FR)**

(72) Inventeur: **Quemin, Eric**
**94340 Joinville le Pont (FR)**

(74) Mandataire: **Boulard, Denis**
**L'OREAL - D.I.P.I.**
**25-29 Quai Aulagnier**
**92600 Asnières (FR)**

(56) Documents cités:
EP-A- 1 093 795          WO-A-02/17875
FR-A- 2 755 852          GB-A- 760 579
GB-A- 2 275 419          US-A- 6 024 968

• NN: "O/W Sprayable Sunscreen - High SPF" UNIQEMA, [Online] 2002, pages 1-2, XP002287326 Uniqema Extrait de l'Internet: URL: http://www.uniqema.com/Uniqema_Product Catalogue/ProdCat/default.asp?Region=&PAGE 1=http://www.uniqema.com/Uniqema_ ProductCa talogue/ProdCat/Formulations.asp? Region=Eu rope&MoveTo=112>

• NN: "O/W Skin Care Lotion (SPF 8)" UNIQEMA, [Online] 2002, pages 1-2, XP002287327 Uniqema Extrait de l'Internet: URL:http: //www.uniqema.com/Uniqema_Product Catalogue/ProdCat/default.asp?Region=&PAGE 1=http://www.uniqema.com/Uniqema_ ProductCa talogue/ProdCat/Formulations.asp? Region=Eu rope&MoveTo=112>

• ANONYMOUS: "Dekorative kosmetische zubereitungen" RESEARCH DISCLOSURE, KENNETH MASON PUBLICATIONS, HAMPSHIRE, GB, vol. 466, no. 49, février 2003 (2003-02), pages 1-7, XP007132213 ISSN: 0374-4353

• NN: "Sedefos RTM 75" GATTEFOSSE, [Online] pages 1-1, XP002287328 Gattefosse Extrait de l'Internet: URL:http://www.gattefosse.com/ pharma/produ cts/sedefo75.htm> & NN: "searched for http://www.gattefosse.com/ pharma/products/ sedefo75.htm" INTERNET ARCHIVE WAYBACK MACHINE, [Online] XP002317516 Extrait de l'Internet: URL:http: //web.archive.org/web/*/http://ww w.gattefosse.com/pharma/products/sedefo75. htm>

Remarques:
Le dossier contient des informations techniques présentées postérieurement au dépôt de la demande et ne figurant pas dans le présent fascicule.

**Description**

La présente invention concerne une composition de maquillage comprenant une nanoémulsion à base d'un système de tensioactifs et des particules solides dispersées.

L'invention se rapporte également à un procédé de préparation de ladite composition et à son utilisation pour le maquillage des matières kératiniques, en particulier la peau.

Les nanoémulsions sont des émulsions huile dans eau dont les globules d'huile ont une granulométrie très fine, c'est-à-dire une taille moyenne en nombre, inférieure à 500 nanomètres (nm). Elles sont généralement fabriquées par fragmentation mécanique d'une phase huileuse dans une phase aqueuse en présence de tensioactifs. Dans le cas des nanoémulsions, la petite taille des globules huileuses est obtenue notamment grâce à au moins un passage dans un homogénéiseur haute pression. La petite taille des globules leur confère des propriétés intéressantes sur le plan cosmétique qui les distinguent des émulsions classiques : elles sont translucides, voire transparentes et présentent une texture originale. Elles peuvent également véhiculer des actifs de façon plus efficace.

On connaît dans l'état de la technique des nanoémulsions comprenant une phase lipidique amphiphile constituée de phospholipides, d'eau et d'huile. Ces émulsions présentent l'inconvénient d'être instables au stockage aux températures traditionnelles de conservation, à savoir entre 0 et 45°C. Elles conduisent à des compositions jaunes et produisent des odeurs de rance qui se développent après quelques jours de conservation.

On connaît également des nanoémulsions stabilisées par un enrobage cristal liquide lamellaire obtenu par l'association d'un tensioactif hydrophile et d'un tensioactif lipophile. Toutefois, ces associations sont délicates à déterminer. De plus, les nanoémulsions obtenues présentent un toucher cireux et filmogène, peu agréable pour l'utilisateur.

La demande EP-A-728.460 décrit des nanoémulsions contenant deux types de tensioactifs :

- un tensioactif non ionique sélectionné parmi les esters gras de polyéthylène glycol ou de sorbitol ;
- un tensioactif ionique, dont le dicétylphosphate de potassium.

La demande EP-A-1093795 décrit des nanoémulsions contenant des pigments inorganiques, des émulsifiants dont la valeur HLB est susceptible de changer selon le pH ou la température, et des agents filmogènes.

La demande WO 02/056843 décrit une nanoémulsion translucide comprenant un système ternaire de tensioactifs, utilisable notamment comme composition de maquillage de la peau. Cette nanoémulsion est obtenue par homogénéisation à haute pression de l'émulsion. Toutefois, l'introduction de particules solides telles que des pigments comme les oxydes de fer ou les dioxydes de titane, ou bien encore des charges dans la nanoémulsion posent plusieurs problèmes. En effet, il n'est pas possible d'introduire les particules solides (pigments ou charges) pendant la fabrication de la nanoémulsion car ces particules endommagent la cellule d'homogénéisation de l'homogénéiseur haute pression. D'autre part, il est nécessaire de broyer le plus finement possible les pigments pour éviter la présence d'amas (ou agrégats) de pigments dans la composition, ces amas de pigments étant nuisibles à l'obtention d'une bonne couleur (couleur développée) du maquillage. Or la technique de broyage n'est pas compatible avec la nanoémulsion car cette dernière est totalement détruite par les effets mécaniques du broyage : il n'est donc pas possible de broyer les pigments avec la nanoémulsion. Par ailleurs, l'introduction directe des pigments ou des charges dans la nanoémulsion fait chuter la viscosité de la nanoémulsion et les pigments et/ou charges sédimentent dans la composition : les particules solides (pigments ou charges) ne sont plus réparties de manière homogène dans la composition ; la composition n'est donc pas stable et est inappropriée comme produit de maquillage puisqu'elle ne permet pas d'obtenir un maquillage homogène. Un besoin existe donc de pouvoir introduire des pigments ou des charges dans une émulsion, notamment une nanoémulsion, de manière satisfaisante.

La présente invention a pour but de fournir une composition de maquillage comprenant une nanoémulsion et des particules solides (notamment pigments, charges) dispersées de manière homogène.

L'invention a également pour but de fournir une composition pigmentée de maquillage comprenant une nanoémulsion, qui soit stable au stockage, notamment après un stockage de deux mois à 45 °C.

Les inventeurs ont découvert qu'il est possible d'obtenir une composition stable de maquillage en introduisant les particules solides (pigments ou charges) préalablement dispersés dans l'eau à l'aide de tensioactifs ioniques particuliers, après la formation de la nanoémulsion.

De façon plus précise, la présente invention a donc pour objet une composition de maquillage comprenant :

i) une émulsion comportant une phase huileuse dispersée dans une phase aqueuse, caractérisée en ce que l'émulsion a des globules d'huile dont la taille moyenne en nombre est inférieure ou égale à 500 nm et qu'elle contient un système de tensioactifs comprenant :

(a) au moins un tensioactif non ionique ; et
(b) au moins un tensioactif ionique ayant une tension interfaciale dynamique inférieure ou égale à 7 millinewton/mètre,

ii) des particules solides dispersées dans la phase aqueuse de l'émulsion et ayant une taille moyenne en nombre inférieure ou égale à 10 μm.

L'invention a encore pour objet un procédé cosmétique de maquillage des matières kératiniques, notamment de la peau, comprenant l'application sur les matières kératiniques, notamment sur la peau, d'une composition selon la revendication 1.

On entend par composition stable une composition stable après un stockage de deux mois à 45 °C, de préférence également stable après un stockage de deux mois à température ambiante (25 °C), et mieux également stable après un stockage de deux mois à 4 °C.

Une composition stable est, au sens de la présente invention, une composition présentant une dispersion homogène des pigments, c'est-à-dire une composition qui ne contient pas d'agrégats de pigments visibles à l'oeil nu lorsque la composition de maquillage est appliquée sur les matières kératiniques, en particulier sur la peau. Le maquillage présente une couleur homogène sans laisser de traces, de trainées de couleur.

De plus, les pigments sont bien répartis dans la composition, permettant ainsi d'obtenir un produit de maquillage présentant une couleur homogène lors de son application sur les matières kératiniques, en particulier sur la peau.

Par ailleurs, les pigments sont incorporés dans la composition tout en préservant les propriétés cosmétiques de la nanoémulsion : la composition présente notamment de bonnes propriétés hydratantes pour la peau.

L'émulsion a des globules d'huile ayant une taille moyenne en nombre inférieure ou égale à 500 nm (notamment allant de 50 nm à 500 nm), préférentiellement inférieure ou égale à 300 nm (notamment allant de 50 nm à 300 nm), plus préférentiellement inférieure ou égale à 200 nm (notamment allant de 50 nm à 200 nm), et notamment allant 50 à 150 nm. Cette taille de globules peut être mesurée par exemple avec un appareil BROOKHAVEN BI 90 et déterminée selon la méthode connue de « Diffusion quasi élastique de la lumière ». La diminution de la taille des globules permet de favoriser la concentration des actifs dans les couches superficielles de la peau (effet véhicule).

Le système de tensioactifs utilisable dans l'émulsion de l'invention comprend, comme indiqué ci-dessus, au moins un tensioactif non ionique (a).

Le tensioactif non ionique peut être présent en une teneur allant de 1 % à 10 % en poids, par rapport au poids total de la composition, et de préférence allant de 2 % à 6 % en poids.

Le tensioactif non ionique peut être choisi parmi les esters gras éthoxylés comportant 8 à 100 unités d'oxyde éthylène, les esters d'acide gras de sorbitane, et leurs mélanges.

Les esters du tensioactif non ionique (a) comporte généralement une chaîne grasse ayant de 16 à 22 atomes de carbone. La chaîne grasse des esters peut être notamment choisie parmi les motifs stéaryle, béhényle, arachidyle, palmityle, cetyle et leurs mélanges tel que cétéaryle.

Le nombre d'unités d'oxyde d'éthylène peut aller de 8 à 100, de préférence de 10 à 80, et mieux de 20 à 60. Selon un mode particulier de réalisation de l'invention, ce nombre est de 40.

A titre d'exemple d'ester gras éthoxylé ayant 40 unités d'oxyde d'éthylène, on peut citer l'ester d'acide stéarique comprenant 40 unités d'oxyde d'éthylène, tel que le produit commercialisé sous la dénomination Myrj 52 (stéarate de polyéthylèneglycol 40 OE ; nom CTFA : PEG-40 stéarate) par la société UNIQEMA.

A titre d'exemple d'ester d'acide gras de sorbitane, on peut citer le tristéarate de sorbitane.

Avantageusement, le tensioactif non ionique (a) est un mélange comprenant au moins un ester gras éthoxylé comportant 8 à 100 unités d'oxyde éthylène, de préférence de 10 à 80 unités d'oxyde éthylène, et au moins un ester d'acide gras de sorbitane.

De préférence, le tensioactif non ionique (a) est un mélange comprenant un ester gras éthoxylé, en particulier le PEG-40 stéarate, et un ester de sorbitane, en particulier le tristéarate de sorbitane. En général, le rapport pondéral de l'ester gras éthoxylé 8 à 100 OE (de préférence de 10 à 80 OE) à l'ester de sorbitane du mélange (a) varie de 0,02 à 100, de préférence de 0,04 à 80.

En général, l'ester gras éthoxylé 8 à 100 OE représente 0,01 à 10 % en poids, de préférence de 0,1 à 5 % en poids, et mieux de 0,5 à 3 %, par rapport au poids total de la composition.

L'ester d'acide gras de sorbitane représente généralement 0,1 % à 10 % en poids, et de préférence 0,5 à 5 % en poids, par rapport au poids total de la composition.

Le système de tensioactifs comprend au moins un tensioactif ionique (b) ayant une tension interfaciale dynamique inférieure ou égale à 7 millinewton/mètre.

La tension interfaciale dynamique d'un tensioactif est déterminée selon le protocole décrit ci-après.

Comme tensioactif ionique ayant une tension interfaciale dynamique inférieure à 7 millinewton/mètre, on peut citer les sels alcalins de cétylphosphate, les sels alcalins de palmitoyl sarcosinate, et leurs mélanges. Les sels alcalins sont par exemple les sels de sodium, les sels de potassium. Les tensioactifs ioniques préférés sont choisis parmi le cétylphosphate de potassium, le palmitoyl sarcosinate de sodium, et leurs mélanges.

En général, le rapport pondéral du constituant (b) au constituant (a) du système de tensioactifs varie de 0,02 à 75, de préférence de 0,02 à 10.

La teneur en tensioactif ionique peut aller de 0,05 à 10 % en poids, et de préférence de 0,2 à 5 % et mieux de 0,5 à 3 % en poids, par rapport au poids total de la composition.

La composition selon l'invention comporte une phase huileuse. Typiquement, le rapport pondéral du système de tensioactifs à la phase huileuse varie de $6.10^{-3}$ à 60, de préférence de 0,4 à 19.

Généralement, la phase huileuse représente de 0,5 % à 40 % en poids par rapport au poids total de la composition, et de préférence de 5 à 30 % en poids.

Avantageusement, la phase huileuse représente au moins 20% en poids par rapport au poids total de la composition, et notamment de 20% à 40% en poids.

La phase huileuse de l'émulsion selon l'invention contient au moins une huile qui peut être choisie parmi les huiles d'origine animale ou végétale, les huiles minérales, les huiles de synthèse, les huiles de silicone, les hydrocarbures notamment aliphatiques, et leurs mélanges. Ces huiles peuvent être polaires ou non polaires, volatiles ou non volatiles.

Parmi les huiles polaires, on peut citer les huiles hydrocarbonées comportant des fonctions ester, éther, acide, alcool ou leurs mélanges, telles que par exemple :

- les huiles végétales hydrocarbonées à forte teneur en triglycéride constituées d'ester d'acide gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépin de raisin ou de cassis, d'onagre, de millet, d'orge, de quinoa, d'olive, de seigle, de carthame, de bancoulier, de passiflore, de rosier muscat ou encore les triglycérides des acides capryliques / capriques comme ceux vendus par la société STEARINERIES DUBOIS ou ceux vendus sous une dénomination MIGLYOL 810, 812 et 818 par la société DYNAMIT NOBEL ;

  - les huiles de synthèse de formule $R^1COOR^2$, dans laquelle $R^1$ représente le reste d'un acide gras supérieur, linéaire ou ramifié, comportant de 7 à 19 atomes de carbone, et $R^2$ représente une chaîne hydrocarbonée ramifiée contenant de 3 à 20 atomes de carbone, comme par exemple l'huile de Purcellin (octanoate de cétostéaryle), l'isononoate d'isononyle, les benzoates d'alkyle (en $C_{12}$ à $C_{15}$) ;
  - les esters et les éthers de synthèse comme le myristate d'isopropyle, le palmitate d'éthyl-2-hexyle, les octanoates, les décanoates ou les ricinoléates d'alcools ou de polyalcools ;
  - les esters hydroxylés comme le lactate d'isostéaryle, le malate de di-isostéaryle, et les esters de pentaérythritol.

Parmi les huiles apolaires, on peut citer :

- les huiles de silicones volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone ; les silicones phénylées, comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates,
- les hydrocarbures ou les hydrocarbures fluorés/ou fluorocarbures, linéaires ou ramifiés d'origine synthétique ou minérale, comme les huiles volatiles, telles que les huiles de paraffine (par exemple les isoparaffines), et les hydrocarbures aliphatiques (par exemple l'isododécane), ou non volatiles et leurs dérivés, la vaseline, les polydécènes, le polyisobutène hydrogéné tel que l'huile de Parléam, le squalane, et leurs mélanges.

La phase huileuse peut également comporter des corps gras autres que les huiles indiquées ci-dessus, tels que un ou plusieurs alcools gras comme les alcools stéarylique, cétylique, béhénique, les acides gras comme les acides stéarylique, palmitique et béhénique, les cires tels que les mono, di-, tri stéarate de glycéryle, les mono- , di- , tri- palmitate de glycéryle, les gommes et leurs mélanges.

Lorsqu'il est présent, cet autre corps gras, de préférence l'alcool cétylique, peut représenter de 2% à 10% en poids, de préférence de 2 à 5% en poids, du poids total de l'émulsion.

La composition selon l'invention comprend des particules solides dispersées dans la phase aqueuse de l'émulsion ayant une faille moyenne en nombre inférieure ou égale à 10 μm.

Les particules solides peuvent être choisies parmi les pigments, les charges, et leurs mélanges. Avantageusement, les particules solides comprennent au moins des pigments.

Les particules solides présentes dans la composition selon l'invention sont dispersées sous forme de particules ayant une taille moyenne en nombre inférieure ou égale à 10 μm (notamment allant de 0,5 μm à 10 μm), de préférence inférieure ou égale à 5 μm (notamment allant de 0,5 μm à 5 μm), et préférentiellement inférieure ou égal à 2 μm

(notamment allant de 0,5 $\mu$m à 2 $\mu$m).

La taille des particules solides dispersés dans la composition peut être mesurée avec un granulomètre de type FPIA 2100 vendu par la société MALVERN. L'échantillon de la composition utilisée pour faire la mesure est dilué dans de l'eau de telle sorte que le granulomètre détecte au plus 30000 particules.

Les particules solides dispersées dans la composition peuvent être présentes en une teneur allant de 1 % à 25 % en poids, par rapport au poids total de la composition, de préférence allant de 2 % à 15 % en poids, préférentiellement allant de 3 % à 10 % en poids, et plus préférentiellement allant de 5% à 10% en poids.

Les pigments peuvent être choisis parmi les pigments minéraux, les pigments organiques, et les pigments composites (c'est-à-dire des pigments à base de matériaux minéraux et/ou organiques).

Les pigments minéraux peuvent être choisis parmi les pigments d'oxyde métallique, le mica recouvert de dioxyde de titane, le mica recouvert d'oxychlorure de bismuth, le mica titane recouvert d'oxyde de fer, le mica-titane recouvert de bleu ferrique, le mica titane recouvert d'oxyde de chrome, et leurs mélanges.

Les pigments d'oxyde métallique sont par exemple les oxydes de fer, le dioxyde de titane, les oxydes de zinc, les oxydes de zirconium, les oxydes de cérium, et leurs mélanges. Les pigments minéraux sont de préférence des pigments d'oxyde métallique.

Avantageusement, les pigments minéraux sont choisis parmi les oxydes de fer, les oxydes de zinc, les oxydes de zirconium, les oxydes de cérium, le mica recouvert de dioxyde de titane, le mica recouvert d'oxychlorure de bismuth, le mica titane recouvert d'oxyde de fer, le mica-titane recouvert de bleu ferrique, le mica titane recouvert d'oxyde de chrome, les pigments organiques et leurs mélanges.

La composition peut comprendre en outre du dioxyde de titane.

Les particules organiques destinées à être enrobées peuvent être par exemple :

- le carmin de cochenille,
- les pigments organiques de colorants azoïques, anthraquinoniques, indigoïdes, xanthéniques, pyréniques, quinoliniques, de triphénylméthane, de fluorane ;
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane. Ces colorants comportent généralement au moins un groupe acide carboxylique ou sulfonique ;
- les pigments mélaniques.

La laque organique peut aussi être supportée par tout support compatible tel qu'un support minéral comme les particules d'alumine, d'argile, de zircone ou d'oxydes métalliques, notamment d'oxyde de zinc ou d'oxyde de titane, de talc, de carbonate de calcium, de sulfate de baryum. De préférence, le support minéral est choisi parmi l'alumine, l'oxyde de titane et le sulfate de barium.

La laque organique peut également être supportée par un support tel que la colophane ou le benzoate d'aluminium.

Parmi les pigments organiques, on peut citer les D&C Blue n°4, D&C Brown n° 1, D&C Green n°5, D&C Green n°6, D&C Orange n°4, D&C Orange n°5 , D&C Orange n°10 , D&C Orange n°11 , D&C Red n°6, D&C Red n°7, D&C Red n°17, D&C Red n°21, D&C Red n°22, D&C Red n°27, D&C Red n°28, D&C Red n°30, D&C Red n°31, D&C Red n°33, D&C Red n°34, D&C Red n°36, D&C Violet n°2, D&C Yellow n°7, D&C Yellow n°8, D&C Yellow n°10, D&C Yellow n°11, FD&C Blue n° 1, FD&C Green n°3, FD&C Red n°40, FD&C Yellow n°5, FD&C Yellow n°6.

Parmi les laques organiques, on peut en particulier citer celles connues sous les dénominations suivantes :

D & C Red n° 2 Aluminium lake
D & C Red n° 3 Aluminium lake
D & C Red n° 4 Aluminium lake
D & C Red n° 6 Aluminium lake
D & C Red n° 6 Barium lake
D & C Red n° 6 Barium/Strontium lake
D & C Red n° 6 Strontium lake
D & C Red n° 6 Potassium lake
D & C Red n° 7 Aluminium lake
D & C Red n° 7 Barium lake
D & C Red n° 7 Calcium lake
D & C Red n° 7 Calcium/strontium lake
D & C Red n° 7 Zirconium lake
D & C Red n° 8 Sodium lake
D & C Red n° 9 Aluminium lake

D & C Red n° 9 Barium lake
D & C Red n° 9 Barium/Strontium lake
D & C Red n° 9 Zirconium lake
D & C Red n° 10 Sodium lake
D & C Red n° 19 Aluminium lake
D & C Red n° 19 Barium lake
D & C Red n° 19 Zirconium lake
D & C Red n° 21 Aluminium lake
D & C Red n° 21 Zirconium lake
D & C Red n° 22 Aluminium lake
D & C Red n° 27 Aluminium lake
D & C Red n° 27 Aluminium/Titanium/Zirconium lake
D & C Red n° 27 Barium lake
D & C Red n° 27 Calcium lake
D & C Red n° 27 Zirconium lake
D & C Red n° 28 Aluminium lake
D & C Red n° 30 lake
D & C Red n° 31 Calcium lake
D & C Red n° 33 Aluminium lake
D & C Red n° 34 Calcium lake
D & C Red n° 36 lake
D & C Red n° 40 Aluminium lake
D & C Blue n° 1 Aluminium lake
D & C Green n° 3 Aluminium lake
D & C Orange n° 4 Aluminium lake
D & C Orange n° 5 Aluminium lake
D & C Orange n° 5 Zirconium lake
D & C Orange n° 10 Aluminium lake
D & C Orange n° 17 Barium lake
D & C Yellow n° 5 Aluminium lake
D & C Yellow n° 5 Zirconium lake
D & C Yellow n° 6 Aluminium lake
D & C Yellow n° 7 Zirconium lake
D & C Yellow n° 10 Aluminium Lake
FD & C Blue n° 1 Aluminium lake
FD & C Red n° 4 Aluminium lake
FD & C Red n° 40 Aluminium lake
FD & C Yellow n° 5 Aluminium lake
FD & C Yellow n° 6 Aluminium lake

Les composés chimiques correspondant à chacun des pigments organiques cités précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande à titre de référence.

Les pigments mélaniques utilisables selon l'invention sont en particulier :

- les pigments mélaniques dérivés de sources naturelles ou synthétiques et qui peuvent être obtenus : (A) par oxydation d'au moins un composé indolique ou indolinique, ou (B) par polymérisation, oxydante ou enzymatique, de précurseurs mélaniques, ou (C) par extraction de la mélanine à partir de substances en contenant, ou (D) par culture de microorganismes. De tels pigments mélaniques sont notamment décrits dans les documents EP-A-518773, WO-A-93/13744 et WO-A-93/13745.

Les pigments peuvent être présents dans la composition en une teneur allant de 0,5 % à 25 % en poids, par rapport au poids total de la composition, et de préférence allant de 1 à 12 % % en poids, et préférentiellement allant de 3 à 8 % en poids.

Les particules solides dispersées dans la phase aqueuse de la composition peuvent être également des charges. Par charges, il faut comprendre des particules de toute forme, incolores ou blanches, minérales ou de synthèse, insolubles dans le milieu de la composition quelle que soit la température à laquelle la composition est fabriquée.

Les charges peuvent être notamment choisies parmi les charges minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), le carbonate de calcium précipité, le carbonate et l'hydro-carbonate de magnésium, l'hydroxyapatite, les microsphères de silice creuses, les microcapsules de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent être présentes dans la composition en une teneur allant de 1 % à 15 % en poids, par rapport au poids total de la composition, et de préférence allant de 1 à 12 % % en poids, et préférentiellement allant de 2 à 8 % en poids.

Les particules solides, notamment les pigments, les charges, sont avantageusement dispersées dans la phase aqueuse de la composition en présence d'un tensioactif ionique (dit aussi tensioactif dispersant) ayant une tension interfaciale dynamique inférieure à 7 millinewton/mètre.

Le tensioactif ionique dispersant est de préférence un tensioactif anionique.

Comme tensioactif ionique dispersant ayant une tension interfaciale dynamique inférieure à 7 millinewton/mètre, on peut citer choisi parmi les sels alcalins de cétylphosphate, les sels alcalins de palmitoyl sarcosinate et leurs mélanges. Les sels alcalins sont par exemple les sels de sodium, les sels de potassium. Les tensioactifs préférés sont choisis parmi le cétylphosphate de potassium, le palmitoyl sarcosinate de sodium, et leurs mélanges.

Avantageusement, les particules solides sont dispersées préalablement avec le tensioactif ionique dispersant ayant une tension interfaciale dynamique inférieure à 7 millinewton/mètre dans une fraction de la phase aqueuse de la composition pour former une pâte pigmentaire, cette dernière étant ajoutée à l'émulsion préparée également préalablement, conformément au procédé de préparation de la composition décrit ultérieurement.

Le tensioactif ionique ayant une tension interfaciale dynamique inférieure à 7 millinewton/mètre permettant de disperser les particules solides est présent dans la composition en une quantité efficace pour permettre d'obtenir une composition pigmentée stable. En pratique, cette quantité peut aller de 0,1 à 3 % en poids, par rapport au poids total de la composition, de préférence allant de 0,3 à 2 % en poids, et préférentiellement allant de 0,5 à 1,8 % en poids.

Avantageusement, la composition selon l'invention peut comprendre au moins un épaississant aqueux non ionique. Cet épaississant favorise la bonne stabilité de la composition.

L'épaississant aqueux non ionique peut être choisi parmi :

- les polymères de cellulose tels que l'hydroxyéthylcellulose, la méthylcellulose, l'hydroxypropylcellulose. Parmi celles-ci, on peut citer notamment les gommes vendues sous la dénomination de "Cellosize QP 4400 H" par la Société Amercol,
- les gommes de guar non-ioniques comprenant des groupements hydroxyalkyle en $C_1$-$C_6$. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120 et JAGUAR HP 105 par la société MEYHALL, ou sous la dénomination GALACTASOL 40H4FD2 par la société AQUALON.
- les carraghénanes ;
- les gommes de caroube, de scléroglucane, de gellane, de rhamsan, de karaya,
- les polymères vinyliques, comme les polyvinylpyrrolidones, les copolymères de l'éther méthylvinylique et de l'anhydride malique, les copolymères de vinylpyrrolidone et d'acétate de vinyle ; les copolymères de vinylpyrrolidone et de caprolactame ; l'alcool polyvinylique ;
- les polymères associatifs et notamment les polyuréthanes associatifs ;
- et leurs mélanges.

L'agent épaississant est de préférence un polyuréthane associatif.

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques. En particulier, ces polymères comportent au moins deux chaînes lipophiles hydrocarbonées, ayant de $C_6$ à $C_{30}$ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.

A titre d'exemple, des polymères associatifs utilisables dans l'invention, on peut citer le polymère $C_{16}$-OE$_{120}$-C$_{16}$ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en $C_{20}$ et à liaison uréthane, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010, le Sérad FX1035 et le Serad 1070 vendus par la société HULS, le Rhéolate 255, le Rhéolate 278 et le Rhéolate 244 vendus par la société RHEOX. On peut aussi utiliser le produit DW 1206F et le DW 1206J, ou bien encore le Borchigel LW 44 de la société BORCHERS.

Les polymères utilisables dans l'invention sont en particulier ceux décrits dans l'article de G. Fonnum, J. Bakke et Fk. Hansen - Colloid Polym. Sci 271, 380.389 (1993).

L'épaississant aqueux non ionique peut être présent dans la composition selon l'invention en une teneur allant de 0,01 % à 5 % en poids, par rapport au poids total de la composition, de préférence allant de 0,01 % à 3 % en poids, et préférentiellement allant de 0,01 % à 1,5 % en poids.

La composition selon l'invention a généralement la consistance d'un gel ou d'une crème. La viscosité de la composition peut aller de 1 à 70 poises (0,1 à 7 Pa.s), de préférence de 5 à 50 poises (0,5 à 5 Pa.s), la viscosité étant mesurée à 25°C avec un viscosimètre Rhéomat 180 (mobile 3).

La composition selon l'invention peut comprendre d'autres ingrédients cosmétiques usuels pouvant être choisis notamment parmi les antioxydants, les parfums, les conservateurs, les neutralisants, les cires, les filtres solaires, les vitamines, les hydratants, les composés auto-bronzants, les actifs antirides.

La composition selon l'invention peut contenir des actifs hydrosolubles ou liposolubles ayant une activité cosmétique ou dermatologique.

Les actifs liposolubles sont dans les globules huileux de l'émulsion, tandis que les actifs hydrosolubles sont dans la phase aqueuse de l'émulsion. On peut citer, à titre d'exemples d'actifs, les vitamines, telles que la vitamine E, la vitamine C, la vitamine A, la vitamine PP et leurs dérivés et, en particulier, leurs esters, les provitamines telles que le panthénol, les humectants et les filtres solaires.

Les compositions selon l'invention peuvent se présenter sous la forme d'un fond de teint, d'un produit de maquillage des lèvres (rouge à lèvres ou gloss), d'un fard à paupières, d'un fard à joues, d'un produit anti-cernes, d'un produit de maquillage du corps, d'un eye-liner, d'un mascara.

L'invention a aussi pour objet un procédé de préparation d'une composition de maquillage telle que définie ci-dessus, procédé comprenant :

i) une première étape de préparation :

a) d'une émulsion, notamment d'une nanoémulsion, en mélangeant la phase aqueuse et la phase huileuse sous agitation vive, à une température allant de 60 à 95°C, puis à effectuer une homogénéisation à une pression allant, de préférence, de $4.10^7$ Pa à $18.10^7$ Pa et notamment allant de $6.10^7$ Pa à $18.10^7$ Pa (homogénéisation haute pression) ;

b) d'une pâte pigmentaire en dispersant dans une phase aqueuse des particules solides, notamment des pigments ou des charges, en présence de tensioactif ionique ayant une tension interfaciale dynamique inférieure à 7 millinewton/mètre;

ii) d'une deuxième étape d'introduction de la pâte pigmentaire dans l'émulsion sous agitation modérée de telle sorte à conserver la structure de l'émulsion de la composition.

La pâte pigmentaire comprend avantageusement de 15 % à 75 % en poids par rapport au poids total de la pâte pigmentaire, de préférence de 30 % à 65 % en poids, de pigments et de 0,5 % à 10 % en poids, de préférence de 3 % à 7 % en poids dudit tensioactif ionique.

Les exemples qui suivent, permettront de mieux comprendre l'invention. Dans les exemples, sauf indication contraire, les pourcentages et parties sont exprimés en poids.

Méthode de mesure de la tension interfaciale dynamique d'un tensioactif :

La tension interfaciale dynamique du tensioactif est mesurée à l'aide d'un tensiomètre, par exemple le tensiomètre vendu sous la dénomination Drop volume tensiometer modèle DVT-10 par la société KRUSS.

Un échantillon d'un mélange eau + tensioactif contenant 0,5 % en poids, par rapport au poids total du mélange, de tensioactif est placé dans un tube au fond duquel est branché un tuyau capillaire, connecté à une seringue de précision contenant un huile (l'huile choisie correspond en général à l'huile majoritaire présente dans la composition selon l'invention ; dans les exemples de composition mentionnés ci-après, l'huile majoritaire est le polyisobutène hydrogéné ou huile de Parléam). Le tube rempli est placé dans un bain marie à 70 °C de telle sorte que le mélange eau + tensioactif n'est pas opaque. Une vis sans fin active la seringue afin de pousser l'huile dans le capillaire à un débit constant de 1 ml/h. A l'orifice du capillaire (orifice d'un diamètre d), une goutte d'huile se forme puis se détache et remonte à la surface du mélange eau + tensioactif.

Une diode électronique luminescente et une photodiode, positionnées sur le tube, détectent les gouttes d'huile qui se détachent du capillaire.

Le temps écoulé entre la formation de 2 gouttes consécutives est mesuré à l'aide d'un chronomètre. Le débit étant constant, le temps est alors converti pour déterminer le volume de chaque goutte, ce volume étant directement dépendant de la tension interfaciale dynamique $\sigma_i$. Cette dernière est alors calculée à l'aide de l'équation suivante :

$$\sigma_i = V(\rho_H - \rho_L)\,g\,/\,\Pi\,d$$

dans laquelle:

$V$ représente le volume de la goutte exprimé en cm$^3$

$\rho_H$ représente la densité du mélange eau + tensioactif, exprimée en g/cm$^3$

$\rho_L$ représente la densité de l'huile, exprimée en g/cm$^3$

$g$ représente l'accélération de la pesanteur et a pour valeur 9,8 m/s$^2$

$d$ représente le diamètre de l'orifice du capillaire exprimé en mètre

$\rho_H$ et $\rho_L$ sont mesurées de façon classique à l'aide d'un densimètre

$\sigma_i$ est exprimé en mN/m.

10 intervalles de temps entre 2 gouttes sont mesurés et le résultat obtenu est une moyenne de ces 10 mesures.

Ce procédé s'applique pour des tensioactifs solubles dans l'eau à la température de 70 °C, c'est-à-dire que le mélange eau + tensioactif n'est pas opaque à 70 °C.

Lorsque le tensioactif est insoluble dans l'eau, le procédé est inversé, c'est-à-dire que le tensioactif est d'abord mélangé à l'huile et la seringue est remplie d'eau.

## Exemples de compositions :

Les nanoémulsions des exemples 1 et 2 ci-dessous ont été obtenues en formant une pré-émulsion grossière au rotor-stator, en ajoutant la phase aqueuse A sur la phase huileuse B, à 80°C. Le prémix était ensuite passé cinq fois dans un homogénéisateur haute pression (Soavi type OBL 20) avec une pression au premier étage de 4 x 10$^7$ Pa (1100 bars) et une pression au second étage de 7 x 10$^6$ Pa (120 bars), avec un refroidissement à 70°C en sortie.

## Exemple 1

On a préparé une nanoémulsion ayant la composition suivante :

| | | |
|---|---|---|
| **A** | eau | 60,05 % |
| | méthylparaben | 0,2 % |
| **B** | Tristéarate de sorbitane (1) | 1 % |
| | Alcool cétylique | 4,7 % |

(suite)

|  | Stéarate de glycéryle (2) | 3,9 % |
|---|---|---|
|  | Stéarate de polyéthylène glycol (40 OE) | 2,22 % |
|  | Polyisobutène hydrogéné (Huile de Parléam) | 13,5 % |
|  | Isononanoate d'isononyle | 13,5 % |
|  | Cétyl phosphate de potassium | 0,83 % |
|  | propylparaben | 0.1 % |

(1) SPAN 65 V de UNIQUEMA
(2) STEARATE DE GLYCERYLE CODEX de Stéarinerie Dubois

## Exemple 2

On a préparé une nanoémulsion ayant la composition suivante :

| A | eau | 60,56 % |
|---|---|---|
|  | méthylparaben | 0,2 % |
| B | Tristéarate de sorbitane (1) | 1,28 % |
|  | Alcool cétylique | 5,72 % |
|  | Stéarate de glycéryle (2) | 4,71 % |
|  | Stéarate de polyéthylène glycol (40 OE) | 2,86 % |
|  | Polyisobutène hydrogéné (Huile de Parléam) | 23,6 % |
|  | Cétyl phosphate de potassium | 1,07 % |

(1) SPAN 65 V de UNIQUEMA
(2) STEARATE DE GLYCERYLE CODEX de Stéarinerie Dubois

## Exemple 3

On a préparé une nanoémulsion ayant la composition suivante :

| A | eau | 60,75 % |
|---|---|---|
|  | méthylparaben | 0,2 % |
| B | Tristéarate de sorbitane (1) | 1 % |
|  | Alcool cétylique | 4 % |
|  | Stéarate de glycéryle (2) | 3,9 % |
|  | Stéarate de polyéthylène glycol (40 OE) | 2,22 % |
|  | Polyisobutène hydrogéné (Huile de Parléam) | 10 % |
|  | Malate de di-isostéaryle | 10 % |
|  | Cétyl phosphate de potassium | 0,83 % |
|  | Propylparabène | 0,1 % |
|  | Diméthicone (3) | 7 % |

(1) SPAN 65 V de UNIQUEMA
(2) STEARATE DE GLYCERYLE CODEX de Stéarinerie Dubois

(3) DOW CORNING DC 200 - 5 cst de DOW CORNING

Les nanoémulsions des exemples 1 à 3 selon l'invention ont la consistance d'un gel et sont stables même après les deux mois de vieillissement accéléré à 4°C, à la température ambiante, et à 45°C.

**Exemple 4 :**

a) On a préparé une pâte pigmentaire ayant la composition suivante :

| | |
|---|---|
| Dioxyde de titane | 20 % |
| Oxydes de fer jaune | 4,7 % |
| Oxydes de fer brun | 4 % |
| Oxydes de fer noir | 1 % |
| Palmitoyl sarcosinate de sodium | 2,5 % |
| Eau | 67,8 % |

On mélange l'ensemble des ingrédients à température ambiante puis le mélange est traité au broyeur à billes pendant 30 minutes à 25 °C à la vitesse de 1200 tours/minute. On obtient alors une pâte pigmentaire stable et homogène.

b) On a préparé un fond de teint ayant la composition suivante :

| | |
|---|---|
| Nanoémulsion de l'exemple 1 | 90 % |
| Pâte pigmentaire selon a) | 10 % |

On disperse la pâte pigmentaire dans la nanoémulsion en agitant à l'aide d'une pâle droite à 400 tour/minute.
On obtient un fond de teint fluide ayant une viscosité de 6,5 poises (0,65 Pa.s) mesurée à 25 °C à l'aide du Rhéomat 180 équipé du mobile n°3, la mesure étant effectuée après 10 minutes de rotation du mobile.
Ce fond de teint est stable au stockage pendant 2 mois à 45 °C, présente une texture légère tant au toucher que lors de son application sur la peau. Après application, le fond de teint est confortable, hydrate et adoucie la peau.

**Exemple 5 :**

a) On a préparé une pâte pigmentaire ayant la composition suivante :

| | |
|---|---|
| Dioxyde de titane | 22,46 % |
| Oxydes de fer jaune | 5,24 % |
| Oxydes de fer brun | 4,5 % |
| Oxydes de fer noir | 1,14 % |
| Palmitoyl sarcosinate de sodium | 2,5 % |
| Eau | 64,16 % |

On mélange l'ensemble des ingrédients à température ambiante puis le mélange est traité au broyeur à billes pendant 30 minutes à 25 °C à la vitesse de 1200 tours/minute. On obtient alors une pâte pigmentaire stable et homogène.

b) On a préparé un fond de teint ayant la composition suivante :

| | |
|---|---|
| Nanoémulsion de l'exemple 2 | 70 % |

(suite)

| | |
|---|---|
| Pâte pigmentaire selon a) | 30 % |

On disperse la pâte pigmentaire dans la nanoémulsion en agitant à l'aide d'une pâle droite à 400 tour/minute.

On obtient un fond de teint fluide ayant une viscosité de 8 poises (0,8 Pa.s) mesurée à 25 °C à l'aide du Rhéomat 180 équipé du mobile n°3, la mesure étant effectuée après 10 minutes de rotation du mobile.

Ce fond de teint est stable au stockage pendant 2 mois à 45 °C, présente une texture légère tant au toucher que lors de son application sur la peau. Après application, le fond de teint est confortable, hydrate et adoucie la peau.

## Exemple 6 :

a) On a préparé une pâte pigmentaire ayant la composition suivante :

| | |
|---|---|
| Dioxyde de titane | 22,46 % |
| Oxydes de fer jaune | 5,24 % |
| Oxydes de fer brun | 4,5 % |
| Oxydes de fer noir | 1,14 % |
| Palmitoyl sarcosinate de sodium | 2,5 % |
| Eau | 64,16 % |

On mélange l'ensemble des ingrédients à température ambiante puis le mélange est traité au broyeur à billes pendant 30 minutes à 25 °C à la vitesse de 1200 tours/minute. On obtient alors une pâte pigmentaire stable et homogène.

b) On a préparé un fond de teint ayant la composition suivante :

| | |
|---|---|
| Nanoémulsion de l'exemple 1 | 86,2 % |
| Pâte pigmentaire selon a) Polyuréthane associatif (Ser-Ad FX 1100 de la société | 13,75 % |
| SERVO DELDEN) | 0,5 % |

On disperse la pâte pigmentaire dans la nanoémulsion en agitant à l'aide d'une pâle droite à 400 tour/minute, puis on ajoute le polyuréthane.

On obtient un fond de teint fluide ayant une viscosité de 9 poises (0,9 Pa.s) mesurée à 25 °C à l'aide du Rhéomat 180 équipé du mobile n°3, la mesure étant effectuée après 10 minutes de rotation du mobile.

Ce fond de teint est stable au stockage pendant 2 mois à 45 °C, présente une texture légère tant au toucher que lors de son application sur la peau. Après application, le fond de teint est confortable, hydrate et adoucie la peau.

## Exemple 7 :

a) On a préparé une pâte pigmentaire ayant la composition suivante :

| | |
|---|---|
| Dioxyde de titane | 6,60 % |
| Oxydes de fer brun, jaune | 12,73 % |
| Oxydes de fer noir | 5,44 % |
| Sel de calcium du rouge lithol B | 12,73 % |
| Palmitoyl sarcosinate de sodium | 5,79 % |
| Eau | 56,71 % |

On mélange l'ensemble des ingrédients à température ambiante puis le mélange est traité au broyeur à billes pendant 30 minutes à 25 °C à la vitesse de 1200 tours/minute. On obtient alors une pâte pigmentaire stable et homogène.

b) On a préparé un rouge à lèvres ayant la composition suivante :

| | |
|---|---|
| Nanoémulsion de l'exemple 3 | 79,5 % |
| Polyuréthane associatif (Ser-Ad FX 1100 de la société SERVO DELDEN) | 0,5 % |
| Eau | 5% |
| Pâte pigmentaire selon a) | 10 % |
| Glycérine | 5 % |

On disperse dans la nanoémulsion la pâte pigmentaire et la glycérine en agitant à l'aide d'une pâle droite à 400 tour/minute, puis on ajoute le mélange d'eau et de polyuréthane.

On obtient un rouge à lèvres fluide ayant une viscosité de 30 poises (3 Pa.s) mesurée à 25 °C à l'aide du Rhéomat 180 équipé du mobile n°3, la mesure étant effectuée après 10 minutes de rotation du mobile.

Ce rouge à lèvres est stable au stockage pendant 2 mois à 45 °C, présente une texture légère lors de son application sur les lèvres.

**Revendications**

1. Composition de maquillage comprenant :

   i) une émulsion comportant une phase huileuse dispersée dans une phase aqueuse, **caractérisée en ce que** l'émulsion a des globules d'huile dont la taille moyenne en nombre est inférieure ou égale à 500 nm et qu'elle contient un système de tensioactifs comprenant :

   (a) au moins un tensioactif non ionique ; et
   (b) au moins un tensioactif ionique ayant une tension interfaciale dynamique inférieure ou égale à 7 millinewton/mètre,

   ii) des particules solides dispersées dans la phase aqueuse de l'émulsion et ayant une taille moyenne en nombre inférieure ou égale à 10 $\mu$m.

2. Composition selon la revendication précédente, **caractérisée en ce qu'**elle est stable après un stockage de deux mois à 45 °C.

3. Composition selon l'une quelconque des revendications précédentes, **caractérisée par le fait que** les particules solides ont une taille moyenne en nombre allant de 0,5 $\mu$m à 10 $\mu$m.

4. Composition selon l'une quelconque des revendications précédentes, **caractérisée par le fait que** les particules solides ont une taille moyenne en nombre allant de 0,5 $\mu$m à 5 $\mu$m.

5. Composition selon l'une quelconque des revendications précédentes, **caractérisée par le fait que** les particules solides ont notamment une taille moyenne en nombre allant de 0,5 $\mu$m à 2 $\mu$m.

6. Composition selon l'une quelconque des revendications précédentes, **caractérisée par le fait que** le tensioactif non ionique est choisi parmi les esters gras éthoxylés comportant 8 à 100 unités d'oxyde d'éthylène, les esters d'acide gras de sorbitane, et leurs mélanges.

7. Composition selon l'une quelconque des revendications précédentes, **caractérisée par le fait que** le tensioactif non ionique comprend au moins un ester gras éthoxylé comportant 8 à 100 unités d'oxyde d'éthylène et au moins un ester d'acide gras de sorbitane.

8. Composition selon l'une quelconque des revendications 6 ou 7, **caractérisée par le fait que** l'ester gras éthoxylé , comprend de 10 à 80 unités d'oxyde d'éthylène.

9.  Composition selon l'une quelconque des revendications 6 à 8, **caractérisée par le fait que** l'ester gras éthoxylé comprend 40 unités d'oxyde d'éthylène,

10. Composition selon l'une quelconque des revendications 6 à 9, **caractérisée par le fait que** l'ester gras éthoxylé est le stéarate de polyéthylène glycol 40 OE.

11. Composition selon l'une quelconque des revendications 6 à 10, **caractérisée par le fait que** l'ester d'acide gras de sorbitane est le tristéarate de sorbitane.

12. Composition selon l'une quelconque des revendications précédentes, **caractérisée par le fait que** le tensioactif non ionique est présent en une teneur allant de 1 % à 10 % en poids, par rapport au poids total de la composition.

13. Composition selon la revendication précédente, **caractérisée par le fait que** le tensioactif non ionique est présent en une teneur allant de 2 % à 6 % en poids.

14. Composition selon l'une quelconque des revendications précédentes, **caractérisée par le fait que** le tensioactif ionique du système tensioactif est choisi parmi les sels alcalins de cétylphosphate et les sels alcalins de palmitoyl sarcosinate.

15. Composition selon l'une quelconque des revendications précédentes, **caractérisée par le fait que** le tensioactif ionique du système tensioactif est choisi parmi le cétyl phosphate de potassium, le palmitoyl sarcosinate de sodium et leurs mélanges.

16. Composition selon l'une quelconque des revendications précédentes, **caractérisée par le fait que** le rapport pondéral du tensioactif ionique (b) au tensioactif non Ionique (a) est tel que :

$$0,02 \leq b/a \leq 75.$$

17. Composition selon l'une quelconque des revendications 6 à 16, **caractérisée par le fait que** le rapport pondéral de l'ester gras éthoxylé à l'ester d'acide gras du sorbitane varie de 0,02 à 100.

18. Composition selon l'une quelconque des revendications 6 à 17, **caractérisée par le fait que** l'ester d'acide gras de sorbitane représente de 0,1% à 10% en poids, du poids total de la composition.

19. Composition selon la revendication précédente, **caractérisée par le fait que** l'ester d'acide gras de sorbitane représente de 0,5 à 5% en poids, du poids total de la composition.

20. Composition selon l'une quelconque des revendications 6 à 19, **caractérisée par le fait que** l'ester gras éthoxylé représente de 0,01 % à 14% en poids.

21. Composition selon la revendication précédente, **caractérisée par le fait que** l'ester gras éthoxylé représente de 0,1 à 5 % en poids, du poids total de la composition.

22. Composition selon l'une quelconque des revendications précédentes, **caractérisée par le fait que** la teneur en tensioactif ionique représente de 0,05% à 10 % en poids,

23. Composition selon la revendication précédente, **caractérisée par le fait que** la teneur en tensioactif ionique représente de 0,2 % à 5 % en poids, du poids total de la composition.

24. Composition selon l'une quelconque des revendications précédentes, **caractérisée par le fait que** l'émulsion a des globules d'huile dont la taille moyenne en nombre est inférieure ou égale à 300 nm.

25. Composition selon l'une quelconque des revendications précédentes, **caractérisée par le fait que** la phase huileuse représente de 0,5 % à 40% en poids, par rapport au poids total de l'émulsion.

26. Composition selon l'une quelconque des revendications précédentes, **caractérisée par le fait que** la phase huileuse représente de 20% à 40% en poids par rapport au poids total de la composition.

27. Composition selon l'une quelconque des revendications précédentes, **caractérisée par le fait que** la phase huileuse comprend une huile choisie parmi les huiles d'origine animale ou végétale, les huiles minérales, les huiles de synthèse, les huiles de silicone, les hydrocarbures aliphatiques, et leurs mélanges.

28. Composition selon l'une quelconque des revendications précédentes, **caractérisée par le fait qu'**elle comprend au moins un autre corps gras choisi parmi les alcools gras, les acides gras, les cires, les gommes et leurs mélanges.

29. Composition selon la revendication précédente, **caractérisée par le fait que** l'autre corps gras représente de 2 à 10 % en poids, du poids total de l'émulsion.

30. Composition selon la revendication 28 ou 29, **caractérisée par le fait que** les alcools gras sont choisis parmi les alcools stéaryliques, cétylique, béhénique, et les acides gras sont choisis parmi les acides stéarique, palmitique et béhénique.

31. Composition selon la revendication précédente, **caractérisée par le fait que** l'alcool gras est l'alcool cétylique.

32. Composition selon la revendication 28 ou 29, **caractérisée par le fait que** l'autre corps gras est choisi parmi les mono, di-, tri stéarate de glycéryle, les mono- , di-, tri- palmitate de glycéryle.

33. Composition selon les revendications précédentes, **caractérisée par le fait que** les particules solides sont choisies parmi les pigments, les charges, et leurs mélanges.

34. Composition selon la revendication précédente, **caractérisée par le fait que** les pigments sont choisis parmi les oxydes métalliques, le mica recouvert de dioxyde de titane, le mica recouvert d'oxychlorure de bismuth, le mica titane recouvert d'oxyde de fer, le mica-titane recouvert de bleu ferrique, le mica titane recouvert d'oxyde de chrome, et leurs mélanges.

35. Composition selon les revendications précédentes, **caractérisée par le fait que** les particules solides sont des pigments d'oxyde métallique.

36. Composition selon l'une quelconque des revendications 33 à 35, **caractérisée par le fait que** les pigments sont choisis parmi les oxydes de fer, le dioxyde de titane, les oxydes de zinc, les oxydes de zirconium, les oxydes de cérium, et leurs mélanges.

37. Composition selon l'une quelconque des revendications précédentes, **caractérisée par le fait que** les particules solides comprennent des pigments organiques choisis parmi :

   - le carmin de cochenille,
   - les pigments organiques de colorants azoïques, anthraquinoniques, indigoïdes, xanthéniques, pyréniques, quinoliniques, de triphénylméthane, de fluorane
   - les laques organiques ;
   - les pigments mélaniques.

38. Composition selon la revendication précédente, **caractérisée par le fait que** la laque est choisi parmi les sels insolubles de sodium, de potassium, de calcium, de baryum, d'aluminium, de zirconium, de strontium, de titane, de colorants organiques acides azoïques, anthraquinoniques, indigoïdes, xanthéniques, pyréniques, quinoliniques, de triphénylméthane, de fluorane.

39. Composition selon l'une des revendications 37 ou 38, **caractérisée par le fait que** la laque organique est supportée par un support minéral.

40. Composition selon l'une quelconque des revendications 33 à 39, **caractérisée par le fait que** les charges sont choisies parmi le talc, le mica, la silice, le kaolin, les poudres de polyamide, les poudres de poly-β-alanine, les poudres de polyéthylène, les poudres de polymères de tétrafluoroéthylène, la lauroyl-lysine, l'amidon, le nitrure de bore, les microsphères creuses de chlorure de polyvinylidène/acrylonitrile, les microsphères creuses de copolymères d'acide acrylique, les microbilles de résine de silicone, le carbonate de calcium précipité, le carbonate et l'hydro-carbonate de magnésium, l'hydroxyapatite, les microsphères de silice creuses, les microcapsules de céramique, les savons métalliques dérivés d'acides organiques carboxyliques ayant de 8 à 22 atomes de carbone.

41. Composition selon l'une quelconque des revendications précédentes, **caractérisée par le fait que** les particules solides dispersées sont présentes en une teneur allant de 1 % à 25 % en poids, par rapport au poids total de la composition.

42. Composition selon la revendication précédente, **caractérisée par le fait que** les particules solides dispersées sont présentes en une teneur allant de 3 % à 10 % en poids, par rapport au poids total de la composition.

43. Composition selon l'une quelconque des revendications 33 à 42, **caractérisée par le fait qu'**elle comprend des pigments en une teneur allant de 0,5 % à 25 % en poids, par rapport au poids total de la composition.

44. Composition selon la revendication précédente, **caractérisée par le fait qu'**elle comprend des pigments en une teneur allant de 1 % à 12 % en poids.

45. Composition selon l'une quelconque des revendications précédentes, **caractérisée par le fait qu'**elle comprend des charges en une teneur allant de 1 % à 15 % en poids, par rapport au poids total de la composition.

46. Composition selon la revendication précédente, **caractérisée par le fait qu'**elle comprend des charges en une teneur allant de 1 % à 12 % en poids.

47. Composition selon l'une quelconque des revendications précédentes, **caractérisée par le fait que** le tensioactif ionique ayant une tension interfaciale dynamique inférieure à 7 millinewton/mètre dispersant les particules solides est présent en une quantité allant de 0,1 % à 3 % en poids, par rapport au poids total de la composition.

48. Composition selon la revendication précédente, **caractérisée par le fait que** le tensioactif ionique ayant une tension interfaciale dynamique inférieure à 7 millinewton/mètre dispersant les particules solides est présent en une quantité allant de 0,3 % à 2 % en poids, par rapport au poids total de la composition.

49. Composition selon l'une quelconque les revendications précédentes, **caractérisée par le fait qu'**elle comprend un épaississant aqueux non ionique.

50. Composition selon la revendication précédente, **caractérisée par le fait que** l'épaississant aqueux non ionique est choisi parmi les polymères de cellulose, les gommes de guar non-ioniques comprenant des groupements hydroxyalkyle en $C_1$-$C_6$ ; les carraghénanes ; les gommes de caroube, de scléroglucane, de gellane, de rhamsan, de karaya ; les polyvinylpyrrolidones, les copolymères de l'éther méthylvinylique et de l'anhydride malique, les copolymères de vinylpyrrolidone et d'acétate de vinyle ; les copolymères de vinylpyrrolidone et de caprolactame ; l'alcool polyvinylique , les polymères associatifs.

51. Composition selon la revendication précédente, **caractérisée par le fait que** l'épaississant aqueux non ionique est choisi parmi les polyuréthanes associatifs.

52. Composition selon l'une quelconque des revendications 49 à 51, **caractérisée par le fait que** l'épaississant aqueux non ionique est présent en une teneur allant de 0,01 % à 5 % en poids, par rapport au poids total de la composition.

53. Composition selon l'une quelconque des revendications précédentes, **caractérisée en ce qu'**elle contient un ingrédient cosmétique usuels choisi parmi les antioxydants, les parfums, les conservateurs, les neutralisants, les cires, les filtres solaires, les vitamines, les hydratants, les composés auto-bronzants, les actifs antirides.

54. Composition selon l'une quelconque des revendications précédentes, **caractérisée par le fait qu'**elle se présente sous la forme d'un fond de teint, d'un produit de maquillage des lèvres, d'un fard à paupières, d'un fard à joues, d'un produit anti-cernes, d'un produit de maquillage du corps, d'un eye-liner, d'un mascara.

55. Procédé cosmétique de maquillage des matières kératiniques, notamment de la peau, comprenant l'application sur les matières kératiniques, notamment sur la peau, d'une composition selon l'une quelconque des revendications 1 à 54.

**56.** Procédé de préparation d'une composition de maquillage selon l'une quelconque des revendications 1 à 54 comprenant :

i) une première étape de préparation :

a) d'une émulsion, notamment d'une nanoémulsion, en mélangeant une phase aqueuse et une phase huileuse sous agitation vive, à une température allant de 60 à 95°C, puis à effectuer une homogénéisation à une pression allant de $6.10^7$ Pa à $18.10^7$Pa.
b) d'une pâte pigmentaire en dispersant dans une phase aqueuse des particules solides en présence de tensioactif ionique ayant une tension interfaciale dynamique inférieure à 7 millinewton/mètre ;

ii) d'une deuxième étape d'introduction de la pâte pigmentaire dans l'émulsion sous agitation modérée maintenant la structure de l'émulsion de la composition.

**Claims**

**1.** Make-up composition comprising:

i) an emulsion comprising an oily phase dispersed in an aqueous phase, **characterized in that** the emulsion has oil globules, the number-average size of which is less than or equal to 500 nm, and **in that** it contains a surfactant system comprising:

(a) at least one non-ionic surfactant; and
(b) at least one ionic surfactant with a dynamic interface tension of less than or equal to 7 millinewtons/metre,

ii) solid particles dispersed in the aqueous phase of the emulsion and having a number-average size of less than or equal to 10 $\mu$m.

**2.** Composition according to the preceding claim, **characterized in that** it is stable after storage for two months at 45°C.

**3.** Composition according to either one of the preceding claims, **characterized in that** the solid particles have a number-average size ranging from 0.5 $\mu$m to 10 $\mu$m.

**4.** Composition according to any one of the preceding claims, **characterized in that** the solid particles have a number-average size ranging from 0.5 $\mu$m to 5 $\mu$m.

**5.** Composition according to any one of the preceding claims, **characterized in that** the solid particles have in particular a number-average size ranging from 0.5 $\mu$m to 2 $\mu$m.

**6.** Composition according to any one of the preceding claims, **characterized in that** the non-ionic surfactant is chosen from ethoxylated fatty esters comprising 8 to 100 ethylene oxide units, sorbitan fatty acid esters, and mixtures thereof.

**7.** Composition according to any one of the preceding claims, **characterized in that** the non-ionic surfactant comprises at least one ethoxylated fatty ester comprising 8 to 100 ethylene oxide units and at least one sorbitan fatty acid ester.

**8.** Composition according to either one of Claims 6 and 7, **characterized in that** the ethoxylated fatty ester comprises from 10 to 80 ethylene oxide units.

**9.** Composition according to any one of Claims 6 to 8, **characterized in that** the ethoxylated fatty ester comprises 40 ethylene oxide units.

**10.** Composition according to any one of Claims 6 to 9, **characterized in that** the ethoxylated fatty ester is polyethylene glycol stearate comprising 40 EO.

**11.** Composition according to any one of Claims 6 to 10, **characterized in that** the sorbitan fatty acid ester is sorbitan tristearate.

12. Composition according to any one of the preceding claims, **characterized in that** the non-ionic surfactant is present at a content ranging from 1% to 10% by weight, relative to the total weight of the composition.

13. Composition according to the preceding claim, **characterized in that** the non-ionic surfactant is present at a content ranging from 2% to 6% by weight.

14. Composition according to any one of the preceding claims, **characterized in that** the ionic surfactant of the surfactant system is chosen from alkali metal salts of cetyl phosphate and alkali metal salts of palmitoyl sarcosinate.

15. Composition according to any one of the preceding claims, **characterized in that** the ionic surfactant of the surfactant system is chosen from potassium cetyl phosphate, sodium palmitoyl sarcosinate, and mixtures thereof.

16. Composition according to any one of the preceding claims, **characterized in that** the weight ratio of the ionic surfactant (b) to the non-ionic surfactant (a) is such that:

$$0{,}02 \leq b/a \leq 75.$$

17. Composition according to any one of Claims 6 to 16, **characterized in that** the weight ratio of the ethoxylated fatty ester to the sorbitan fatty acid ester ranges from 0.02 to 100.

18. Composition according to any one of Claims 6 to 17, **characterized in that** the sorbitan fatty acid ester represents from 0.1% to 10% by weight, of the total weight of the composition.

19. Composition according to the preceding claim, **characterized in that** the sorbitan fatty acid ester represents from 0.5% to 5% by weight, of the total weight of the composition.

20. Composition according to any one of Claims 6 to 19, **characterized in that** the ethoxylated fatty ester represents from 0.01% to 10% by weight.

21. Composition according to the preceding claim, **characterized in that** the ethoxylated fatty ester represents from 0.1% to 5% by weight, of the total weight of the composition.

22. Composition according to any one of the preceding claims, **characterized in that** the content of ionic surfactant represents from 0.05% to 10% by weight.

23. Composition according to the preceding claim, **characterized in that** the content of ionic surfactant represents from 0.2% to 5% by weight, of the total weight of the composition.

24. Composition according to any one of the preceding claims, **characterized in that** the emulsion has oil globules, the number-average size of which is less than or equal to 300 nm.

25. Composition according to any one of the preceding claims, **characterized in that** the oily phase represents from 0.5% to 40% by weight, relative to the total weight of the emulsion.

26. Composition according to any one of the preceding claims, **characterized in that** the oily phase represents from 20% to 40% by weight, relative to the total weight of the composition.

27. Composition according to any one of the preceding claims, **characterized in that** the oily phase comprises an oil chosen from oils of animal or plant origin, mineral oils, synthetic oils, silicone oils, aliphatic hydrocarbons, and mixtures thereof.

28. Composition according to any one of the preceding claims, **characterized in that** it comprises at least one other fatty substance chosen from fatty alcohols, fatty acids, waxes, gums, and mixtures thereof.

29. Composition according to the preceding claim, **characterized in that** the other fatty substance represents from 2% to 10% by weight, of the total weight of the emulsion.

30. Composition according to Claim 28 or 29, **characterized in that** the fatty alcohols are chosen from stearyl alcohol, cetyl alcohol and behenyl alcohol, and the fatty acids are chosen from stearic acid, palmitic acid and behenic acid.

31. Composition according to the preceding claim, **characterized in that** the fatty alcohol is cetyl alcohol.

32. Composition according to Claim 28 or 29, **characterized in that** the other fatty substance is chosen from glyceryl monostearate, glyceryl distearate, glyceryl tristearate, glyceryl monopalmitate, glyceryl dipalmitate and glyceryl tripalmitate.

33. Composition according to the preceding claims, **characterized in that** the solid particles are chosen from pigments, fillers, and mixtures thereof.

34. Composition according to the preceding claim, **characterized in that** the pigments are chosen from metal oxides, mica coated with titanium dioxide, mica coated with bismuth oxychloride, titanium mica coated with iron oxide, titanium mica coated with ferric blue, titanium mica coated with chromium oxide, and mixtures thereof.

35. Composition according to the preceding claims, **characterized in that** the solid particles are metal oxide pigments.

36. Composition according to any one of Claims 33 to 35, **characterized in that** the pigments are chosen from iron oxides, titanium dioxide, zinc oxides, zirconium oxides, cerium oxides, and mixtures thereof.

37. Composition according to any one of the preceding claims, **characterized in that** the solid particles comprise organic pigments chosen from:

    - cochenille carmine,
    - organic pigments of azo, anthraquinone, indigoid, xanthene, pyrene, quinoline, triphenylmethane or fluorane dyes;
    - organic lakes;
    - melanin pigments.

38. Composition according to the preceding claim, **characterized in that** the lake is chosen from insoluble salts of sodium, potassium, calcium, barium, aluminium, zirconium, strontium or titanium, or of acidic organic azo, anthraquinone, indigoid, xanthene, pyrene, quinoline, triphenylmethane and fluorane dyes.

39. Composition according to either of Claims 37 and 38, **characterized in that** the organic lake is supported by a mineral support.

40. Composition according to any one of Claims 33 to 39, **characterized in that** the fillers are chosen from talc, mica, silica, kaolin, polyamide powders, poly-$\beta$-alanine powders, polyethylene powders, tetrafluoroethylene polymer powders, lauroyllysine, starch, boron nitride, hollow microspheres of polyvinylidene chloride/acrylonitrile, hollow microspheres of acrylic acid copolymers, silicone resin microbeads, precipitated calcium carbonate, magnesium carbonate, magnesium hydrogen carbonate, hydroxyapatite, hollow silica microspheres, ceramic microcapsules, and metal soaps derived from organic carboxylic acids containing from 8 to 22 carbon atoms.

41. Composition according to any one of the preceding claims, **characterized in that** the dispersed solid particles are present at a content ranging from 1% to 25% by weight, relative to the total weight of the composition.

42. Composition according to the preceding claim, **characterized in that** the dispersed solid particles are present at a content ranging from 3% to 10% by weight, relative to the total weight of the composition.

43. Composition according to any one of Claims 33 to 42, **characterized in that** it comprises pigments at a content ranging from 0.5% to 25% by weight, relative to the total weight of the composition.

44. Composition according to the preceding claim, **characterized in that** it comprises pigments at a content ranging from 1% to 12% by weight.

45. Composition according to any one of the preceding claims, **characterized in that** it comprises fillers at a content ranging from 1% to 15% by weight, relative to the total weight of the composition.

**46.** Composition according to the preceding claim, **characterized in that** it comprises fillers at a content ranging from 1% to 12% by weight.

**47.** Composition according to any one of the preceding claims, **characterized in that** the ionic surfactant with a dynamic interface tension of less than 7 millinewtons/metre dispersing the solid particles is present in an amount ranging from 0.1% to 3% by weight, relative to the total weight of the composition.

**48.** Composition according to the preceding claim, **characterized in that** the ionic surfactant with a dynamic interface tension of less than 7 millinewtons/metre dispersing the solid particles is present in an amount ranging from 0.3% to 2% by weight, relative to the total weight of the composition.

**49.** Composition according to any one of the preceding claims, **characterized in that** it comprises a non-ionic aqueous thickener.

**50.** Composition according to the preceding claim, **characterized in that** the non-ionic aqueous thickener is chosen from cellulose polymers, non-ionic guar gums comprising $C_1$-$C_6$ hydroxyalkyl groups, carrageenans, carob gum, scleroglucan gum, gellan gum, rhamsan gum, karaya gum, polyvinylpyrrolidones, copolymers of methyl vinyl ether and of malic anhydride, copolymers of vinylpyrrolidone and of vinyl acetate, copolymers of vinylpyrrolidone and of caprolactam, polyvinyl alcohol and associative polymers.

**51.** Composition according to the preceding claim, **characterized in that** the non-ionic aqueous thickener is chosen from associative polyurethanes.

**52.** Composition according to any one of Claims 49 to 51, **characterized in that** the non-ionic aqueous thickener is present at a content ranging from 0.01% to 5% by weight, relative to the total weight of the composition.

**53.** Composition according to any one of the preceding claims, **characterized in that** it contains a common cosmetic ingredient chosen from antioxidants, fragrances, preserving agents, neutralizers, waxes, sunscreens, vitamins, moisturizers, self-tanning compounds and antiwrinkle active agents.

**54.** Composition according to any one of the preceding claims, **characterized in that** it is in the form of a foundation, a lip make-up product, an eye shadow, a face powder, a concealer product, a body make-up product, an eyeliner or a mascara.

**55.** Cosmetic process for making up keratin materials, in particular the skin, comprising the application to the keratin materials, in particular to the skin, of a composition according to any one of Claims 1 to 54.

**56.** Process for preparing a make-up composition according to any one of Claims 1 to 54, comprising:

i) a first step of preparing:

a) an emulsion, in particular a nanoemulsion, by mixing an aqueous phase and an oily phase with vigorous stirring, at a temperature ranging from 60°C to 95°C, and then homogenizing at a pressure ranging from $6 \times 10^7$ Pa to $18 \times 10^7$ Pa;
b) a pigmentary paste, by dispersing solid particles in an aqueous phase in the presence of an ionic surfactant with a dynamic interface tension of less than 7 millinewtons/metre;

ii) a second step of introducing the pigmentary paste into the emulsion with moderate stirring, conserving the structure of the emulsion of the composition.

**Patentansprüche**

**1.** Zusammensetzung zum Schminken, die enthält:

i) eine Emulsion, die eine in einer wässrigen Phase dispergierte Ölphase enthält, **dadurch gekennzeichnet, dass** die Emulsion Öltröpfchen aufweist, deren zahlenmittlere Größe höchstens 500 nm beträgt, und **dadurch**, dass sie ein System von grenzflächenaktiven Stoffen enthält, umfassend:

(a) mindestens einen nichtionischen grenzflächenaktiven Stoff; und

(b) mindestens einen ionischen grenzflächenaktiven Stoff mit einer dynamischen Grenzflächenspannung von höchstens 7 Millinewton/Meter,

ii) feste Partikel, die in der wässrigen Phase der Emulsion dispergiert sind und eine zahlenmittlere Größe von höchstens 10 μm aufweisen.

2. Zusammensetzung nach dem vorhergehenden Anspruch, **dadurch gekennzeichnet, dass** sie nach zweimonatiger Aufbewahrung bei 45 °C stabil ist.

3. Zusammensetzung nach einem der vorhergehenden Ansprüche, **dadurch gekennzeichnet, dass** die festen Partikel eine zahlenmittlere Größe von 0,5 bis 10 μm aufweisen.

4. Zusammensetzung nach einem der vorhergehenden Ansprüche, **dadurch gekennzeichnet, dass** die festen Partikel eine zahlenmittlere Größe von 0,5 bis 5 μm aufweisen.

5. Zusammensetzung nach einem der vorhergehenden Ansprüche, **dadurch gekennzeichnet, dass** die festen Partikel insbesondere eine zahlenmittlere Größe von 0,5 bis 2 μm aufweisen.

6. Zusammensetzung nach einem der vorhergehenden Ansprüche, **dadurch gekennzeichnet, dass** der nichtionische grenzflächenaktive Stoff unter den ethoxylierten Fettsäureestern mit 8 bis 100 Ethylenoxideinheiten, Sorbitanfettsäureestern und deren Gemischen ausgewählt ist.

7. Zusammensetzung nach einem der vorhergehenden Ansprüche, **dadurch gekennzeichnet, dass** der nichtionische grenzflächenaktive Stoff mindestens einen ethoxylierten Fettsäureester mit 8 bis 100 Ethylenoxideinheiten und mindestens einen Sorbitanfettsäureester umfasst.

8. Zusammensetzung nach einem der Ansprüche 6 oder 7, **dadurch gekennzeichnet, dass** der ethoxylierte Fettsäureester 10 bis 80 Ethylenoxideinheiten aufweist.

9. Zusammensetzung nach einem der Ansprüche 6 bis 8, **dadurch gekennzeichnet, dass** der ethoxylierte Fettsäureester 40 Ethylenoxideinheiten aufweist.

10. Zusammensetzung nach einem der Ansprüche 6 bis 9, **dadurch gekennzeichnet, dass** der ethoxylierte Fettsäureester das Polyethylenglycol(40 EO)stearat ist.

11. Zusammensetzung nach einem der Ansprüche 6 bis 10, **dadurch gekennzeichnet, dass** es sich bei dem Sorbitanfettsäureester um das Sorbitantristearat handelt.

12. Zusammensetzung nach einem der vorhergehenden Ansprüche, **dadurch gekennzeichnet, dass** der nichtionische grenzflächenaktive Stoff in einer Menge von 1 bis 10 Gew.-%, bezogen auf das Gesamtgewicht der Zusammensetzung, enthalten ist.

13. Zusammensetzung nach dem vorhergehenden Anspruch, **dadurch gekennzeichnet, dass** der nichtionische grenzflächenaktive Stoff in einer Menge von 2 bis 6 Gew.-% enthalten ist.

14. Zusammensetzung nach einem der vorhergehenden Ansprüche, **dadurch gekennzeichnet, dass** der ionische grenzflächenaktive Stoff des Tensidsystems unter den Alkalisalzen von Cetylphosphat und den Alkalisalzen von Palmitoylsarcosinat ausgewählt ist.

15. Zusammensetzung nach einem der vorhergehenden Ansprüche, **dadurch gekennzeichnet, dass** der ionische grenzflächenaktive Stoff des Tensidsystems unter Kaliumcetylphosphat, Natriumpalmitoylsarcosinat und deren Gemischen ausgewählt ist.

16. Zusammensetzung nach einem der vorhergehenden Ansprüche, **dadurch gekennzeichnet, dass** das Gewichtsverhältnis des ionischen grenzflächenaktiven Stoffes (b) und des nichtionischen grenzflächenaktiven Stoffes (a) so ist, dass:

$$0,02 \leq b/a \leq 75.$$

17. Zusammensetzung nach einem der Ansprüche 6 bis 16, **dadurch gekennzeichnet, dass** das Gewichtsverhältnis des ethoxylierten Fettsäureesters und des Sorbitanfettsäureesters im Bereich von 0,02 bis 100 liegt.

18. Zusammensetzung nach einem der Ansprüche 6 bis 17, **dadurch gekennzeichnet, dass** der Sorbitanfettsäureester 0,1 bis 10 Gew.-% des Gesamtgewichts der Zusammensetzung ausmacht.

19. Zusammensetzung nach dem vorhergehenden Anspruch, **dadurch gekennzeichnet, dass** der Sorbitanfettsäureester 0,5 bis 5 Gew.-% des Gesamtgewichts der Zusammensetzung ausmacht.

20. Zusammensetzung nach einem der Ansprüche 6 bis 19, **dadurch gekennzeichnet, dass** der ethoxylierte Fettsäureester 0,01 bis 10 Gew.-% ausmacht.

21. Zusammensetzung nach dem vorhergehenden Anspruch, **dadurch gekennzeichnet, dass** der ethoxylierte Fettsäureester 0,1 bis 5 Gew.-% des Gesamtgewichts der Zusammensetzung ausmacht.

22. Zusammensetzung nach einem der vorhergehenden Ansprüche, **dadurch gekennzeichnet, dass** der Mengenanteil des ionischen grenzflächenaktiven Stoffes 0,05 bis 10 Gew.-% ausmacht.

23. Zusammensetzung nach dem vorhergehenden Anspruch, **dadurch gekennzeichnet, dass** der Mengenanteil des ionischen grenzflächenaktiven Stoffes 0,2 bis 5 Gew.-% des Gesamtgewichts der Zusammensetzung ausmacht.

24. Zusammensetzung nach einem der vorhergehenden Ansprüche, **dadurch gekennzeichnet, dass** die Emulsion Ölkügelchen enthält, deren zahlenmittlere Größe höchstens 300 nm beträgt.

25. Zusammensetzung nach einem der vorhergehenden Ansprüche, **dadurch gekennzeichnet, dass** die Ölphase 0,5 bis 40 Gew.-%, bezogen auf das Gesamtgewicht der Emulsion, ausmacht.

26. Zusammensetzung nach einem der vorhergehenden Ansprüche, **dadurch gekennzeichnet, dass** die Ölphase 20 bis 40 Gew.-%, bezogen auf das Gesamtgewicht der Zusammensetzung, ausmacht.

27. Zusammensetzung nach einem der vorhergehenden Ansprüche, **dadurch gekennzeichnet, dass** die Ölphase ein Öl enthält, das unter den Ölen tierischer oder pflanzlicher Herkunft, Mineralölen, synthetischen Ölen, Siliconölen, aliphatischen Kohlenwasserstoffen und deren Gemischen ausgewählt ist.

28. Zusammensetzung nach einem der vorhergehenden Ansprüche, **dadurch gekennzeichnet, dass** sie mindestens eine weitere Fettsubstanz enthält, die unter den Fettalkoholen, Fettsäuren, Wachsen, Gummis und deren Gemischen ausgewählt ist.

29. Zusammensetzung nach dem vorhergehenden Anspruch, **dadurch gekennzeichnet, dass** die weitere Fettsubstanz 2 bis 10 Gew.-% des Gesamtgewichts der Emulsion ausmacht.

30. Zusammensetzung nach Anspruch 28 oder 29, **dadurch gekennzeichnet, dass** die Fettalkohole unter Stearylalkohol, Cetylalkohol und Behenylalkohol ausgewählt sind und die Fettsäuren unter Stearinsäure, Palmitinsäure und Behensäure ausgewählt sind.

31. Zusammensetzung nach dem vorhergehenden Anspruch, **dadurch gekennzeichnet, dass** es sich bei dem Fettalkohol um Cetylalkohol handelt.

32. Zusammensetzung nach Anspruch 28 oder 29, **dadurch gekennzeichnet, dass** die weitere Fettsubstanz unter Glycerylmono-, -di- und -tristearat und Glycerylmono-, -di- und -tripalmitat ausgewählt ist.

33. Zusammensetzung nach den vorhergehenden Ansprüchen, **dadurch gekennzeichnet, dass** die festen Partikel unter den Pigmenten, Füllstoffen und deren Gemischen ausgewählt sind.

34. Zusammensetzung nach dem vorhergehenden Anspruch, **dadurch gekennzeichnet, dass** die Pigmente unter den Metalloxiden, mit Titandioxid beschichteten Glimmerpigmenten, mit Bismutoxidchlorid beschichteten Glimmerpigmenten, mit Eisenoxid beschichteten Titan-Glimmer-Pigmenten, mit Eisenblau beschichteten Titan-Glimmer-Pigmenten, mit Chromdioxid beschichteten Titan-Glimmer-Pigmenten und deren Gemischen ausgewählt sind.

35. Zusammensetzung nach den vorhergehenden Ansprüchen, **dadurch gekennzeichnet, dass** die festen Partikel Metalloxidpigmente sind.

36. Zusammensetzung nach einem der Ansprüche 33 bis 35, **dadurch gekennzeichnet, dass** die Pigmente unter den Eisenoxiden, Titandioxid, Zinkoxiden, Zirconiumoxiden, Ceroxiden und deren Gemischen ausgewählt sind.

37. Zusammensetzung nach einem der vorhergehenden Ansprüche, **dadurch gekennzeichnet, dass** die festen Partikel organische Pigmente umfassen, die ausgewählt sind unter:

    - Cochenille-Karmin;
    - organischen Pigmenten von Azofarbstoffen, Anthrachinon-Farbstoffen, Indigoiden, Xanthen-Farbstoffen, Pyren-Farbstoffen, Chinolin-Farbstoffen, Triphenylmethan-Farbstoffen und Fluoran-Farbstoffen;
    - organischen Lacken;
    - Melaninpigmenten.

38. Zusammensetzung nach dem vorhergehenden Anspruch, **dadurch gekennzeichnet, dass** der Lack unter den unlöslichen Salzen von Natrium, Kalium, Calcium, Barium, Aluminium, Zirconium, Strontium oder Titan von organischen sauren Azofarbstoffen, Anthrachinon-Farbstoffen, Indigoiden, Xanthen-Farbstoffen, Pyren-Farbstoffen, Chinolin-Farbstoffen, Triphenylmethan-Farbstoffen und Fluoran-Farbstoffen ausgewählt ist.

39. Zusammensetzung nach einem der Ansprüche 37 oder 38, **dadurch gekennzeichnet, dass** der organische Lack von einem anorganischen Trägermaterial getragen wird.

40. Zusammensetzung nach einem der Ansprüche 33 bis 39, **dadurch gekennzeichnet, dass** die Füllstoffe ausgewählt sind unter Talk, Glimmer, Kieselsäure, Kaolin, Polyamidpulvern, Poly-β-alaninpulvern, Polyethylenpulvern, Pulvern von Tetrafluorethylenpolymeren, Lauroyllysin, Stärke, Bornitrid, Mikrohohlkugeln von Polyvinylidenchlorid/Acrylnitril, Mikrohohlkugeln von Acrylsäurecopolymeren, Siliconharzmikrokugeln, gefälltem Calciumcarbonat, Magnesiumcarbonat, Magnesiumhydrogencarbonat, Hydroxyapatit, Siliciumoxidmikrohohlkugeln, Keramikmikrokapseln, von organischen Carbonsäuren mit 8 bis 22 Kohlenstoffatomen abgeleiteten Metallseifen.

41. Zusammensetzung nach einem der vorhergehenden Ansprüche, **dadurch gekennzeichnet, dass** die dispergierten festen Partikel in einer Menge von 1 bis 25 Gew.-%, bezogen auf das Gesamtgewicht der Zusammensetzung, enthalten sind.

42. Zusammensetzung nach dem vorhergehenden Anspruch, **dadurch gekennzeichnet, dass** die dispergierten festen Partikel in einer Menge von 3 bis 10 Gew.-%, bezogen auf das Gesamtgewicht der Zusammensetzung, enthalten sind.

43. Zusammensetzung nach einem der Ansprüche 33 bis 42, **dadurch gekennzeichnet, dass** sie Pigmente in einer Menge von 0,5 bis 25 Gew.-%, bezogen auf das Gesamtgewicht der Zusammensetzung, enthält.

44. Zusammensetzung nach dem vorhergehenden Anspruch, **dadurch gekennzeichnet, dass** sie Pigmente in einer Menge von 1 bis 12 Gew.-% enthält.

45. Zusammensetzung nach einem der vorhergehenden Ansprüche, **dadurch gekennzeichnet, dass** sie Füllstoffe in einer Menge von 1 bis 15 Gew.-%, bezogen auf das Gesamtgewicht der Zusammensetzung, enthält.

46. Zusammensetzung nach dem vorhergehenden Anspruch, **dadurch gekennzeichnet, dass** sie Füllstoffe in einer Menge von 1 bis 12 Gew.-% enthält.

47. Zusammensetzung nach einem der vorhergehenden Ansprüche, **dadurch gekennzeichnet, dass** der ionische grenzflächenaktive Stoff, der eine dynamische Grenzflächenspannung unter 7 Millinewton/ Meter aufweist und die festen Partikel dispergiert, in einer Menge von 0,1 bis 3 Gew.-%, bezogen auf das Gesamtgewicht der Zusammensetzung, enthalten ist.

**48.** Zusammensetzung nach dem vorhergehenden Anspruch, **dadurch gekennzeichnet, dass** der ionische grenzflächenaktive Stoff, der eine dynamische Grenzflächenspannung unter 7 Millinewton/Meter aufweist und die festen Partikel dispergiert, in einer Menge von 0,3 bis 2 Gew.-%, bezogen auf das Gesamtgewicht der Zusammensetzung, enthalten ist.

**49.** Zusammensetzung nach einem der vorhergehenden Ansprüche, **dadurch gekennzeichnet, dass** sie ein nichtionisches wässriges Verdickungsmittel enthält.

**50.** Zusammensetzung nach dem vorhergehenden Anspruch, **dadurch gekennzeichnet, dass** das nichtionische wässrige Verdickungsmittel unter den Cellulosepolymeren, nichtionischen Guargummen, die $C_{1-6}$-Hydroxyalkylgruppen aufweisen; Carrageenanen; Johannisbrotkernmehl, Skleroglucanen, Gellan, Rhamsan, Karaya-Gummi; Polyvinylpyrrolidonen, Copolymeren von Methylvinylether und Maleinsäureanhydrid, Copolymeren von Vinylpyrrolidon und Vinylacetat; Copolymeren von Vinylpyrrolidon und Caprolactam; Polyvinylalkohol; assoziativen Polymeren ausgewählt ist.

**51.** Zusammensetzung nach dem vorhergehenden Anspruch, **dadurch gekennzeichnet, dass** das nichtionische wässrige Verdickungsmittel unter den assoziativen Polyurethanen ausgewählt ist.

**52.** Zusammensetzung nach einem der Ansprüche 49 bis 51, **dadurch gekennzeichnet, dass** das nichtionische wässrige Verdickungsmittel in einer Menge von 0,01 bis 5 Gew.-%, bezogen auf das Gesamtgewicht der Zusammensetzung, vorliegt.

**53.** Zusammensetzung nach einem der vorhergehenden Ansprüche, **dadurch gekennzeichnet, dass** sie einen üblichen kosmetischen Bestandteil enthält, der unter den Antioxidantien, Parfums, Konservierungsmitteln, Neutralisationsmitteln, Wachsen, Sonnenschutzfiltern, Vitaminen, Hydratisierungsmitteln, Selbstbräunungsmitteln und Wirkstoffen gegen Falten ausgewählt ist.

**54.** Zusammensetzung nach einem der vorhergehenden Ansprüche, **dadurch gekennzeichnet, dass** sie als Makeup, Produkt zum Schminken der Lippen, Lidschatten, Wangenrouge, Produkt gegen Augenringe, Produkt zum Schminken des Körpers, Eyeliner, Mascara vorliegt.

**55.** Kosmetisches Verfahren zum Schminken von Keratinsubstanzen und insbesondere der Haut, das das Aufbringen einer Zusammensetzung nach einem der Ansprüche 1 bis 54 auf die Keratinsubstanzen und insbesondere die Haut umfasst.

**56.** Verfahren zur Herstellung einer Zusammensetzung zum Schminken nach einem der Ansprüche 1 bis 54 mit den folgenden Schritten:

i) einem ersten Schritt der Herstellung:

a) einer Emulsion und insbesondere einer Nanoemulsion, indem eine wässrige Phase und eine Ölphase unter kräf-tigem Rühren bei einer Temperatur von 60 bis 75 °C vermischt werden und anschließend bei einem Druck von $6 \cdot 10^7$ bis $18 \cdot 10^7$ Pa eine Homogenisierung durchgeführt wird;
b) einer Pigmentpaste, indem feste Partikel in Gegenwart eines ionischen grenzflächenaktiven Stoffes mit einer dynamischen Grenzflächenspannung unter 7 Millinewton/Meter in einer wässrigen Phase dispergiert werden;

ii) einem zweiten Schritt, bei dem die Pigmentpaste unter mäßigem Rühren in die Emulsion eingebracht wird, wobei die Struktur der Emulsion der Zusammensetzung beibehalten wird.

## RÉFÉRENCES CITÉES DANS LA DESCRIPTION

**Documents brevets cités dans la description**

- EP 728460 A
- EP 1093795 A
- WO 02056843 A
- EP 518773 A
- WO 9313744 A
- WO 9313745 A

**Littérature non-brevet citée dans la description**

- International Cosmetic Ingredient Dictionnary and Handbook. The Cosmetic, Toiletry, and Fragrance Association, 1997, 371-386524-528
- **G. FONNUM ; J. BAKKE ; FK. HANSEN.** *Colloid Polym. Sci,* 1993, vol. 271, 380-389